# EUROPEAN PATENT SPECIFICATION

(11) **EP 1 670 918 B1**
(45) Date of publication and mention of the grant of the patent: **11.11.2009**
(21) Application number: 04787205.6
(22) Date of filing: 24.09.2004
(51) Int. Cl.: C12N 15/62, C12N 15/58, C12N 5/10, C12P 21/02

(54) **LEADER SEQUENCES FOR USE IN PRODUCTION OF PROTEINS**
LEITSEQUENZEN ZUR VERWENDUNG BEI DER PRODUKTION VON PROTEINEN
SEQUENCES LEADER POUR LA PRODUCTION DE PROTEINES

(30) Priority: 26.09.2003 EP 03103580; 29.09.2003 US 506939 P
(43) Date of publication of application: 21.06.2006
(73) Proprietor: Merck Serono SA, 1267 Coinsins, Vaud (CH)
(72) Inventor: DUPRAZ, Philippe, CH-1023 Crissier (CH); KOBR, Michel, CH-1026 Echandens (CH)
(74) Representative: Merck Serono SA - Geneva Intellectual Property
(86) International application number: PCT/EP2004/052302
(87) International publication number: WO 2005/030963

(56) References cited:
- WO-A-99/53059
- DATABASE EMBL [Online] 17 July 2003 (2003-07-17), ASHKENAZI A.J. ET AL.: "Secretion of glycosylated protein with the use of tissue plasminogen activator prosequence" XP002267400 retrieved from EBI Database accession no. BD599618 & JP 2002 511264 T 16 April 2002 (2002-04-16)
- DATABASE GENESEQ [Online] 7 November 2000 (2000-11-07), MARTIN F.H. ET AL.: "Native mouse IgG1 signal peptide" XP002267401 retrieved from EBI Database accession no. AAY95831 -& WO 00/47741 A (AMGEN INC) 17 August 2000 (2000-08-17)
- DATABASE GENESEQ [Online] 5 December 2000 (2000-12-05), PATEL S ET AL.: "Mouse IgH signal peptide" XP002266931 retrieved from EBI Database accession no. AAY96081 & WO 00/52158 A (MITOTIX INC; CELL GENESYS INC (US)) 20 September 2000 (2000-09-20)

### SEQUENCE LISTING

<110> Applied Research Systems ARS Holding N.V.
   <120> Novel leader sequences for use in production of proteins
   <130> 884 WO
<160> 58
<170> PatentIn version 3.1
<210> 1
   <211> 29
   <212> PRT
   <213> Artificial
<220>
   <221> source
   <223> /note="Description of artificial sequence: IgSP-tPA pre-propeptide"
<220>
   <221> SIGNAL
   <222> (1)..(19)
   <223> murine immunoglobulin signal peptide
<220>
   <221> PROPEP
   <222> (20)..(29)
   <223> human tissue plasminogen activator propeptide
<400> 1
   Met Lys Cys Ser Trp Val Ile Phe Phe Leu Met Ala Val Val Thr Gly
   1 5 10 15
   Val Asn Ser Ser Gln Glu Ile His Ala Arg Phe Arg Arg
   20 25
<210> 2
   <211> 35
   <212> PRT
   <213> Homo sapiens
<400> 2
   Met Asp Ala Met Lys Arg Gly Leu Cys Cys Val Leu Leu Leu Cys Gly
   1 5 10 15
   Ala Val Phe Val Ser Pro Ser Gln Glu Ile His Ala Arg Phe Arg Arg
   20 25 30
   Gly Ala Arg
   35
<210> 3
   <211> 19
   <212> PRT
   <213> Mus musculus
<220>
   <221> SIGNAL
   <222> (1) .. (19)
   <223>
<400> 3
   Met Lys Cys Ser Trp Val Ile Phe Phe Leu Met Ala Val Val Thr Gly
   1 5 10 15
   Val Asn Ser
<210> 4
   <211> 26
   <212> PRT
   <213> Homo sapiens
<220>
   <221> SIGNAL
   <222> (1)..(26)
   <223>
<400> 4
   Met Ala Thr Gly Ser Arg Thr Ser Leu Leu Leu Ala Phe Gly Leu Leu
   1 5 10 15
   Cys Leu Pro Trp Leu Gln Glu Gly Ser Ala
   20 25
<210> 5
   <211> 17
   <212> PRT
   <213> Homo sapiens
<220>
   <221> SIGNAL
   <222> (1) .. (17)
   <223>
<400> 5
   Met Leu Leu Leu Leu Leu Leu Leu Gly Leu Arg Leu Gln Leu Ser Leu
   1 5 10 15
   Gly
<210> 6
   <211> 87
   <212> DNA
   <213> Artificial
<220>
   <221> source
   <223> /note="Description of artificial sequence: nucleic acid encoding IgSP-tPA pre-propeptide"
<400> 6
   atgaagtgca gctgggtgat cttcttcctg atggccgtgg tgaccggcgt gaattccagc 60
   caggagatcc acgccaggtt ccgcaga 87
<210> 7
   <211> 105
   <212> DNA
   <213> Homo sapiens
<400> 7
   atggacgcca tgaagcgcgg cctgtgctgc gtgctgctgc tgtgcggcgc cgtgttcgtg 60
   agccccagcc aggagatcca cgccaggttc cgcagaggcg ccaga 105
<210> 8
   <211> 57
   <212> DNA
   <213> Mus musculus
<400> 8
   atgaagtgca gctgggtgat cttcttcctg atggccgtgg tgaccggcgt gaattcc 57
<210> 9
   <211> 161
   <212> PRT
   <213> Homo sapiens
<400> 9
   Asp Ser Val Cys Pro Gln Gly Lys Tyr Ile His Pro Gln Asn Asn Ser
   1               5                   10                  15
   Ile Cys Cys Thr Lys Cys His Lys Gly Thr Tyr Leu Tyr Asn Asp Cys
               20                  25                  30
   Pro Gly Pro Gly Gln Asp Thr Asp Cys Arg Glu Cys Glu Ser Gly Ser
           35                  40                  45
   Phe Thr Ala Ser Glu Asn His Leu Arg His Cys Leu Ser Cys Ser Lys
       50                  55                  60
   Cys Arg Lys Glu Met Gly Gln Val Glu Ile Ser Ser Cys Thr Val Asp
   65                  70                  75                  80
   Arg Asp Thr Val Cys Gly Cys Arg Lys Asn Gln Tyr Arg His Tyr Trp
                   85                  90                  95
   Ser Glu Asn Leu Phe Gln Cys Phe Asn Cys Ser Leu Cys Leu Asn Gly
               100                 105                 110
   Thr Val His Leu Ser Cys Gln Glu Lys Gln Asn Thr Val Cys Thr Cys
           115                 120                 125
   His Ala Gly Phe Phe Leu Arg Glu Asn Glu Cys Val Ser Cys Ser Asn
       130                 135                 140
   Cys Lys Lys Ser Leu Glu Cys Thr Lys Leu Cys Leu Pro Gln Ile Glu
   145                 150                 155                 160
   Asn
<210> 10
   <211> 239
   <212> PRT
   <213> Homo sapiens
<400> 10
<210> 11
   <211> 34
   <212> DNA
   <213> Artificial
<220>
   <221> source
   <223> /note="Description of artificial sequence: primer"
<400> 11
   tgctctagag cgtcacccct agagtcgagc tgtg 34
<210> 12
   <211> 59
   <212> DNA
   <213> Artificial
<220>
   <221> source
   <223> /note="Description of artificial sequence: primer"
<400> 12
   ggcgttgagc ggccgcggtt catgacgcta gcaccgaatt cacccctgta accactgcc 59
<210> 13
   <211> 51
   <212> DNA
   <213> Artificial
<220>
   <221> source
   <223> /note="Description of artificial sequence: primer"
<400> 13
   cgcagatctg gtctcgcatg aagtgcagct gggtgatctt cttcctgatg g 51
<210> 14
   <211> 43
   <212> DNA
   <213> Artificial
<220>
   <221> source
   <223> /note="Description of artificial sequence: primer"
<400> 14
   ccgtggtgac cggcgtgaat tccgacagcg tgtgccctca ggg 43
<210> 15
   <211> 52
   <212> DNA
   <213> Artificial
<220>
   <221> source
   <223> /note="Description of artificial sequence: primer"
<400> 15
   caagtacatc caccctcaga acaacagcat ctgctgcacc, aagtgccaca ag 52
<210> 16
   <211> 26
   <212> DNA
   <213> Artificial
<220>
   <221> source
   <223> /note="Description of artificial sequence: primer"
<400> 16
   ggcacctacc tgtacaacga ctgccc 26
<210> 17
   <211> 51
   <212> DNA
   <213> Artificial
<220>
   <221> source
   <223> /note="Description of artificial sequence: primer"
<400> 17
   gggcagtcgt tgtacaggta ggtgcccttg tggcacttgg tgcagcagat g 51 <210> 18
   <211> 48
   <212> DNA
   <213> Artificial
<220>
   <221> source
   <223> /note="Description of artificial sequence: primer"
<400> 18
   ctgttgttct gagggtggat gtacttgccc tgagggcaca cgctgtcg 48
<210> 19
   <211> 48
   <212> DNA
   <213> Artificial
<220>
   <221> source
   <223> /note="Description of artificial sequence: primer"
<400> 19
   gaattcacgc cggtcaccac ggccatcagg aagaagatca cccagctg 48
<210> 20
   <211> 25
   <212> DNA
   <213> Artificial
<220>
   <221> source
   <223> /note="Description of artificial sequence: primer"
<400> 20
   cacttcatgc gagaccagat ctgcg 25
<210> 21
   <211> 47
   <212> DNA
   <213> Artificial
<220>
   <221> source
   <223> /note="Description of artificial.sequence: primer"
<400> 21
   ccacaccatg gccaccggca gccgcaccag cctgctgctg gccttcg 47
<210> 22
   <211> 50
   <212> DNA
   <213> Artificial
<220>
   <221> source
<223> /note="Description of artificial sequence: primer"
<400> 22
   gcctgctgtg cctgccctgg ctgcaggagg gcagcgccga cagcgtgtgc 50
<210> 23
   <211> 53
   <212> DNA
   <213> Artificial
<220>
   <221> source
   <223> /note="Description of artificial sequence: primer"
<400> 23
   cctcagggca agtacatcca ccctcagaac aacagcatct gctgcaccaa gtg 53
<210> 24
   <211> 52
   <212> DNA
   <213> Artificial
<220>
   <221> source
   <223> /note="Description of artificial sequence: primer"
<400> 24
   ccacaagggc acctacctgt acaacgactg ccctggccct ggccaggaca cc 52
<210> 25
   <211> 52
   <212> DNA
   <213> Artificial
<220>
   <221> source
   <223> /note="Description of artificial sequence: primer"
<400> 25
   gactgccgcg agtgcgagag cggcagcttc accgccagcg agaaccacct gc 52
<210> 26
   <211> 55
   <212> DNA
   <213> Artificial
<220>
   <221> source
   <223> /note="Description of artificial sequence: primer"
<400> 26
   gccactgcct gagctgcagc aagtgccgca aggagatggg ccaggtggag atcag 55
<210> 27
   <211> 25
   <212> DNA
   <213> Artificial
<220>
   <221> source
   <223> /note="Description of artificial sequence: primer"
<400> 27
   cagctgcacc gtggaccgcg acacc 25
<210> 28
   <211> 53
   <212> DNA
   <213> Artificial
<220>
   <221> source
   <223> /note="Description of artificial sequence: primer"
<400> 28
   ggtgtcgcgg tccacggtgc agctgctgat ctccacctgg cccatctcct tgc 53
<210> 29
   <211> 53
   <212> DNA
   <213> Artificial
<220>
   <221> source
   <223> /note="Description of artificial sequence: primer"
<400> 29
   ggcacttgct gcagctcagg cagtggcgca ggtggttctc gctggcggtg aag 53
<210> 30
   <211> 53
   <212> DNA
   <213> Artificial
<220>
   <221> source
   <223> /note="Description of artificial sequence: primer"
<400> 30
   ctgccgctct cgcactcgcg gcagtcggtg tcctggccag ggccagggca gtc 53
<210> 31
   <211> 56
   <212> DNA
   <213> Artificial
<220>
   <221> source
   <223> /note="Description of artificial sequence: primer"
<400> 31
   gttgtacagg taggtgccct tgtggcactt ggtgcagcag atgctgttgt tctgag 56
<210> 32
   <211> 49
   <212> DNA
   <213> Artificial
<220>
   <221> source
   <223> /note="Description of artificial sequence: primer"
<400> 32
   ggtggatgta cttgccctga gggcacacgc tgtcggcgct gccctcctg 49
<210> 33
   <211> 47
   <212> DNA
   <213> Artificial
<220>
   <221> source
   <223> /note="Description of artificial sequence: primer"
<400> 33
   cagccagggc aggcacagca ggccgaaggc cagcagcagg ctggtgc 47
<210> 34
   <211> 23
   <212> DNA
   <213> Artificial
<220>
   <221> source
   <223> /note="Description of artificial sequence: primer"
<400> 34
   ggctgccggt ggccatggtg tgg 23
<210> 35
   <211> 49
   <212> DNA
   <213> Artificial
<220>
   <221> source
   <223> /note="Description of artificial sequence: primer"
<400> 35
   gctcgagatc tggtctcgca tgctgctgct gctgctgctg ctgggcctg 49
<210> 36
   <211> 41
   <212> DNA
   <213> Artificial
<220>
   <221> source
   <223> /note="Description of artificial sequence: primer"
<400> 36
   aggctccagc tctccctggg cgacagcgtg tgccctcagg g 41
<210> 37
   <211> 43
   <212> DNA
   <213> Artificial
<220>
   <221> source
   <223> /note="Description of artificial sequence: primer"
<400> 37
   gcccagggag agctggagcc tcaggcccag cagcagcagc agc 43
<210> 38
   <211> 52
   <212> DNA
   <213> Artificial
<220>
   <221> source
   <223> ./note="Description of artificial sequence: primer"
<400> 38
   caagtacatc caccctcaga acaacagcat ctgctgcacc aagtgccaca ag 52
<210> 39
   <211> 26
   <212> DNA
   <213> Artificial
<220>
   <221> source
   <223> /note="Description of artificial sequence: primer"
<400> 39
   ggcacctacc tgtacaacga ctgccc 26
<210> 40
   <211> 51
   <212> DNA
   <213> Artificial
<220>
   <221> source
   <223> /note="Description of artificial sequence: primer"
<400> 40
   gggcagtcgt tgtacaggta ggtgcccttg tggcacttgg tgcagcagat g 51
<210> 41
   <211> 47
   <212> DNA
   <213> Artificial
<220>
   <221> source
   <223> /note="Description of artificial sequence: primer"
<400> 41
   ctgttgttct gagggtggat gtacttgccc tgagggcaca cgctgtc 47
<210> 42
   <211> 51
   <212> DNA
   <213> Artificial
<220>
   <221> source
   <223> /note="Description of artificial sequence: primer"
<400> 42
   cgcagatctg gtctcgcatg aagtgcagct gggtgatctt cttcctgatg g 51
<210> 43
   <211> 52
   <212> DNA
   <213> Artificial
<220>
   <221> source
   <223> /note="Description of artificial sequence: primer"
<400> 43
   caagtacatc caccctcaga acaacagcat ctgctgcacc aagtgccaca ag 52
<210> 44
   <211> 26
   <212> DNA
   <213> Artificial
<220>
   <221> source
   <223> /note="Description of artificial sequence: primer"
<400> 44
   ggcacctacc tgtacaacga ctgccc 26
<210> 45
   <211> 51
   <212> DNA
   <213> Artificial
<220>
   <221> source
   <223> /note="Description of artificial sequence: primer"
<400> 45
   gggcagtcgt tgtacaggta ggtgcccttg tggcacttgg tgcagcagat g 51
   <210> 46
   <211> 47
   <212> DNA
   <213> Artificial
<220>
   <221> source
   <223> /note="Description of artificial sequence: primer"
<400> 46
   ctgttgttct gagggtggat gtacttgccc tgagggcaca cgctgtc 47
<210>. 47
   <211> 40
   <212> DNA
   <213> Artificial
<220>
   <221> source
   <223> /note="Description of artificial sequence: primer"
<400> 47
   tctggcgcct ctgcggaacc tggcgtggat ctcctggctg 40
<210> 48
   <211> 48
   <212> DNA
   <213> Artificial
<220>
   <221> source
   <223> /note="Description of artificial sequence: primer"
<400> 48
   gaattcacgc cggtcaccac ggccatcagg aagaagatca cccagctg 48
<210> 49
   <211> 43
   <212> DNA
   <213> Artificial
<220>
   <221> source
   <223> /note="Description of artificial sequence: primer"
<400> 49
   ccgtggtgac cggcgtgaat tccagccagg agatccacgc cag 43
<210> 50
   <211> 486
   <212> DNA
   <213> Homo sapiens
<400> 50
   gacagcgtgt gccctcaggg caagtacatc caccctcaga acaacagcat ctgctgcacc 60
   aagtgccaca agggcaccta cctgtacaac gactgccctg gccctggcca ggacaccgac 120
   tgccgcgagt gcgagagcgg cagcttcacc gccagcgaga accacctgcg ccactgcctg 180
   agctgcagca agtgccgcaa ggagatgggc caggtggaga tcagcagctg caccgtggac 240
   cgcgacaccg tgtgcggctg ccgcaagaac cagtaccgcc actactggag cgagaacctg 300
   ttccagtgct tcaactgcag cctgtgcctg aacggcaccg tgcacctgag ctgccaggag 360
   aagcagaaca ccgtgtgcac ctgccacgcc ggcttcttcc tgcgcgagaa cgagtgcgtg 420
   agctgcagca actgcaagaa gagcctggag tgcaccaagc tgtgcctgcc tcagatcgag 480
   aactaa 486
<210> 51
   <211> 19
   <212> DNA
   <213> Artificial
<220>
   <221> source
   <223> /note="Description of artificial sequence: primer"
<400> 51
   ctagcaaaat aggctgtcc 19
<210> 52
   <211> 34
   <212> DNA
   <213> Artificial
<220>
   <221> source
   <223> /note="Description of artificial sequence: primer"
<400> 52
   gttccgcaga gacagcgtgt gccctcaggg caag 34
<210> 53
   <211> 31
   <212> DNA
   <213> Artificial
<220>
   <221> source
   <223> /note="Description of artificial sequence: primer"
<400> 53
   cacgctgtct ctgcggaacc tggcgtggat c 31
<210> 54
   <211> 53
   <212> DNA
   <213> Artificial
<220>
   <221> source
   <223> /note="Description of artificial sequence: primer"
<400> 54
   ctgccgctct cgcactcgcg gcagtcggtg tcctggccag ggccagggca gtc 53
<210> 55
   <211> 53
   <212> DNA
   <213> Artificial
<220>
   <221> source
   <223> /note="Description of artificial sequence: primer"
<400> 55
   ctgctgctgt gcggcgccgt gttcgtgagc cccagccagg agatccacgc cag 53
<210> 56
   <211> 53
   <212> DNA
   <213> Artificial
<220>
   <221> source
   <223> /note="Description of artificial sequence: primer"
<400> 56
   ctgccgctct cgcactcgcg gcagtcggtg tcctggccag ggccagggca gtc 53
<210> 57
   <211> 57
   <212> DNA
   <213> Artificial
<220>
   <221> source
   <223> /note="Description of artificial sequence: primer"
<400> 57
   gggctcacga acacggcgcc gcacagcagc agcacgcagc acaggccgcg cttcatg 57
<210> 58
   <211> 30
   <212> DNA
   <213> Artificial
<220>
   <221> source
   <223> /note="Description of artificial sequence: primer"
<400> 58
   gccaccatgg acgccatgaa gcgcggcctg 30

## Description

### FIELD OF THE INVENTION

This Invention relates to leader sequences for production of proteins. More specifically, the invention relates to DNA constructs encoding leader sequences comprising an immunoglobulin signal peptide fused to a tissue-type Plasminogen activator propeptide. The invention further relates to the use of these DNA constructs for producing proteins In mammalian cells.

### BACKGROUND

### 1. Processing of protein precursor

Secreted proteins are expressed initially inside the cell In a precursor form containing a leader sequence ensuring entry into the secretory pathway. Such leader sequences, named signal peptides, direct the expressed product across the membrane of the endoplasmic reticulum (ER). Signal peptide are generally cleaved off by signal peptidases during translocation to the ER. Once entered in the secretory pathway, the protein is transported to the Golgl apparatus. From the Golgl the protein can follow different routes that lead to compartments such as the cell vacuole or the cell membrane, or it can be routed out of the cell to be secreted to the external medium (Pfeffer and Rothman (1987) Ann.Rev.Biochem. 58:829-852).

For industrial production of a secreted protein, the protein to be produced needs to be secreted efficiently from the host cell or the host organism. The signal peptide may be, e.g.. the native signal peptide of the protein to be produced, a heterologous signal peptide, or a hybrid of native and heterologous signal peptide. Numerous signal peptides are used for production of secreted proteins. One of them is a murine immunoglobulin signal peptide (IgSP. EMBL Accession No. M13331). IgSP was first identified In 1983 by Loh et al. (Cell. 33:85-93). IgSP Is known to give a good expression In mammalian cells. For example. EP patent No. 0382762 discloses a method of producing horseradish peroxidase by constructing a fusion polypeptide between IgSP and horseradish peroxidase, and WO 00/47741 discloses the recombinant expression of leptin in COS and/or CHO cells using various signal sequences, among others the mouse IgF1 signal sequence.

However, several problems are encountered with the use of currently known signal peptides. One problem often encountered when producing a human protein from a non -human host cell or organism is that the native signal peptide does not ensure efficient translocation and/or cleavage of the signal peptide. This leads to low rates of protein secretion and/or to secretion of mature proteins that display N -terminal extensions due to an incorrect cleavage of the signal peptide. Thus the choice of the signal peptide **is** of great importance for industrial production of a protein.

In addition of leader sequences directing the secretion of the protein, a precursor form can comprise supplemental leader sequences that are cleaved during maturation. These supplemental leader peptides, named propeptides, usually follow the signal peptide. Virtually all peptide hormones, numerous bioactive proteins (for example, growth factors, receptors and cell-adhesion molecules), and many bacterial toxins and viral envelope glycoproteins comprise a propeptide that is post-translationally excised to generate the mature and biologically active protein (Seldah and Chretien (1999) Brain Res. 848:45-62).

Propeptides are cleaved off by enzymes named proprotein convertases. Mammalian proprotein convertases include, e.g., the subtilisin convertases PCSK1, PCSK2 and furin. Furin Is ubiquitously expressed and located in the trans-Golgl network. Furin proteolytically activates large numbers of proproteins substrates in secretory pathway compartments. (Thomas (2002) Nat Rev Mol Cell Biol. 3:753-766). More specifically, furin localizes to the Trans GolgI Network - a late GolgI structure that is responsible for sorting secretory pathway proteins to their final destinations, including the cell surface, endosomes, lysosomes and secretory granules. The site that furin cleaves has been extensively studied. The cleavage site Is positioned after the carboxyl-terminal arginine of the consensus sequence R-X-L/R-R, wherein X may represent any amino acid (Nakayama (1997) Biochem. J 327:625-635). The cleavage efficiency is increased when X is a lysine, a valine, an isoleucine or an alanine (Watanabe et al (1992) J Biol. Chem. 287:8270-8274).

### 2. The tissue-lype plasminogen activator precursor

The human tissue-type plasminogen activator precursor (tPA, SwissProt Acession No. P00750) is synthesized as a precursor form of 562 amino acids comprising a leader sequence of 35 amino acids. This leader sequence comprises a signal peptide of 23 amino acids followed by a propeptide of 12 amino acids.

Köhne et al. (1999, J. Cell. Biochem. 75:446-481) showed that the tPA leader sequence of 35 amino acids was able to rescue intracellular transport of a chimeric Tumor Necrosis Factor Receptor - Immunoglobulin protein (TNFR-Ig) in which all N-linked glycosylation sites had been deleted. In 1999, Etcheverry et al. reported that a leader sequence of 13 amino acids, which comprised the last amino acid of the signal peptide and the entire propeptide of tPA, was able to enhance secretion of a TNFR-Ig fusion when inserted between the TNFR native signal peptide and the TNFR-Ig polypeptide (Etcheverry et al., ESACT meeting, abstract 01.07/P1.02).

WO 99/53059 discloses the use of a chimeric leader sequence comprising the signal sequence of TNFR1 and the propeptide of tPA for expressing the TNFR1-IgG1 protein.

Thus protein processing is a fundamental process for efficient protein secretion, and the choice of the leader sequence is a critical step when producing a secreted polypeptide. In many cases, the leader sequence leads to a low level of secretion or no secretion at all, or to an incorrect or incomplete proteolytic processing. It is therefore the object of the present invention to provide leader sequences that ensure a more efficient secretion and/or processing of polypeptides.

### SUMMARY OF THE INVENTION

The present invention is based on the finding that a leader sequence comprising an immunoglobulin signal peptide fused to a tissue-type plasminogen activator propeptide allows a more efficient secretion and processing of proteins of interest than other known leader sequences. In addition, it has been found that a leader sequence comprising a truncated form of the human tPA propeptide, wherein the carboxyl-terminal extremity of the tPA propeptide consists of amino acids Arg-Xaa-Arg-Arg, allows an efficient secretion and processing of proteins of interest

Therefore, a first aspect of the invention relates to a DNA construct comprising a sequence encoding an IgSP-tPA pre-propeptide comprising an immunoglobulin signal peptide fused to a tPA propeptide consisting of amino acids 23 to 32 of SEQ ID NO:2.

A second aspect relates to a host cell transformed with a DNA construct according to the invention.

A third aspect relates to a process for the production of a polypeptide of Interest comprising the step of transecting a host cell with a DNA construct in accordance with the invention.

A fourth aspect relates to a process for the production of a polypeptide of interest comprising the step of culturing a host cell of the invention.

A fifth aspect relates to the use of a DNA construct in accordance with the invention for producing a polypeptide of interest.

A sixth aspect relates to a fusion polypeptide encoded by a DNA construct in accordance with the invention.

### BRIEF DESCRIPTION OF THE FIGURES

Figure 1 shows an alignment between an IgSP pre-propeptide in accordance with the invention (SEQ ID NO: 1) and the native tPA pre-propeptide (SEQ ID NO: 2).
Figure 2 shows a scheme of the pGL3-GH-TBP-1380 vector used to construct the diff erent signal peptide fused to the TBPI protein.
Figure 3 shows a scheme of the pEF1-GH-TBP-1403 vector used to express the leader peptides-TBPI fusion proteins in transient transfection assays.
Figure 4 shows the amount of TBPI protein detected in supernatant versus cytoplasm of cells transfected with the indicated constructs. Lane GH_SP: TBPI fused to the Growth hormone signal peptide; Lane SEAP_SP: TBPI fused to the secreted alkaline phosphatase signal peptide: Lane IgSP: TBPI fused to the murine immunoglobulin IgG µ-heavy chain signal peptide; Lane IgSP-tPA: TBPI fused to an IgSP pre-propeptide in accordance with the invention.
Figure 5 corresponds to a scheme of the CMV-UbB-LUC-1433 vector used to express the leader peptides-TBPI fusion proteins in stable transfection assays.
Figure 6 shows the amont of TBPI protein detected in the supernatant of pools of clones transfected with IgSP-tPA or with tPA-tPA pre-propeptides fused to TBPI. Pools were maintained eitheir in puromycin and neomycin co-selection (neo/puro) or in puromycin minus Hypoxantine-Tymidine co-selection (HT/puro). Open box and dark box represent two different pulses of 48hrs at 37 °C in medium with 10% FCS. Stripped or squared box represent two pulses of 48hrs at 32 °C in serum-free medium.

### BRIEF DESCRIPTION OF THE SEQUENCES OF THE SEQUENCE LISTING

SEQ ID NO: 1 corresponds to the protein sequence of an IgSP-tPA pre-propeptide in accordance with the invention.
SEQ ID NO: 2 corresponds to the protein sequence of the human tPA pre -propeptide.
SEQ ID NO: 3 corresponds to the protein sequence of the murine IgG µ-heavy chain signal peptide.
SEQ ID NO: 4 corresponds to the protein sequence of the human growth hormone signal peptide.
SEQ ID NO: 5 corresponds to the protein sequence of the human secreted alkaline phosphatase signal peptide.
SEQ ID NO: 6 corresponds to the nucleic sequence of an IgSP-tPA pre-propeptide in accordance with the invention.
SEQ ID NO: 7 corresponds to the nucleic sequence of the human tPA pre-propeptide.
SEQ ID NO: 8 corresponds to the nucleic sequence of the murine IgG µ-heavy chain signal peptide.
SEQ ID NO: 9 corresponds to the protein sequence of the soluble portion of the TNF receptor p55.
SEQ ID NO: 10 corresponds to the protein sequence of mature interferon gamma receptor chain.
SEQ ID Nos. 11 to 20 correspond to primers used to construct and amplify the Ig µ-heavy chain signal peptides.
SEQ ID NO: 21 to 34 correspond to primers used to construct and amplify the human growth hormone signal peptide.
Seq ID NO: 35 to 41 correspond to primers used to construct and amplify the human secreted alkaline phosphatase signal peptide.
Seq ID NO: 42 to 49 correspond to primers used to construct and amplify the IgSP-tPA pre-propeptide in accordance with this invention.
Seq ID NO: 50: correspond to the nucleic sequence of the soluble extracellular portion of the p55 Tumor necrosis factor.
Seq ID NO: 51 to 54 correspond to primers used to introduce a deletion of three amino -acids Into the IgSP-tPA and tPA pre-propeptides in accordance with this invention.
Seq ID NO: 55 to 58 correspond to primers used to generate the tPA -pre-propeptide.

### DETAILED DESCRIPTION OF THE INVENTION

The present invention stems from the finding that leader sequences comprising an immunoglobulin signal peptide fused to a tissue-type plasminogen activator propeptide allow a more efficient secretion and processing of proteins than other known leader sequences. As shown In examples 2 and 3, leader sequences of the present Invention are at least 2 fold more efficient In promoting secretion of proteins of Interest than prior art leader sequences.

In addition, It has been found that a leader sequence comprising a truncated form of the human tPA propeptide, wherein the carboxyl-terminal extremity of the tPA propeptide consists of amino acids Arg-Xaa-Arg-Arg, allows an efficient secretion and processing of proteins of Interest.

Accordingly, the present invention provides novel leader sequences comprising an immunoglobulin signal peptide fused to a tissue-type plasminogen activator propeptide consisting of amino acids 23 to 32 of SEQ ID NO:2. The use of these leader sequences for producing proteins of interest in mammalian cells is a further aspect of the present invention.

A first aspect of the present invention relates to a DNA construct comprising a sequence encoding an IgSP-tPA pre-propeptide comprising an immunoglobulin signal peptide fused to a tissue-type plasminogen activator propeptide consisting of amino acids 23 to 32 of SEQ ID NO:2.

As used herein, the term "signal peptide" refers to a leader sequence ensuring entry Into the secretory pathway. As used herein, the term "propeptide" refers to a leader sequence that follows a signal peptide. As used herein, the term "pre-propeptide" refers to a leader sequence comprising a signal peptide and a propeptide. As used herein, the term "leader sequence" refers to a sequence located at the amino terminal end of the precursor form of a protein. Leader sequences are cleaved off during maturation.

As further used herein, the term "IgSP-tPA pre-propeptide" refers to a leader sequence according to the present invention that comprises an immunoglobulin signal peptide fused to a tissue-type plasminogen activator propeptide.

As shown in example 1, IgSP-tPA pre-propeptides ensures a more efficient secretion of the soluble portion of the TNF receptor p55 (TBPI) than various signal peptides fused directly to TBPI. Example 2 demonstrates that the IgSP-tPA pre-propeptide is more efficient In promoting TBPI secretion than a tPA pre-propeptide alone. Example 3 demonstrates that the IgSP-tPA pre-propeptide is more efficient in promoting secretion of the mature interferon gamma receptor chain (IFNAR) than the native IFNAR signal peptide. Accordingly, IgSP -tPA pre-propeptides ensure a more efficient secretion of polypeptides of interest than any known leader sequence.

Numerous immunoglobulin (Ig) signal peptides from different species are known and are all encompassed within the scope of the present invention. In a preferred embodiment, the Ig signal peptide is a murine immunoglobulin signal peptide. Preferably, the murine Ig signal peptide is a murine IgG µ-heavy chain signal peptide of SEQ ID NO: 3.

Disclosed herein is a sequence encoding a tissue-type plasminogen activator (tPA) propeptide of any origin. For example, a sequence encoding a tPA propeptide of human, murine, rat or bovin origin.

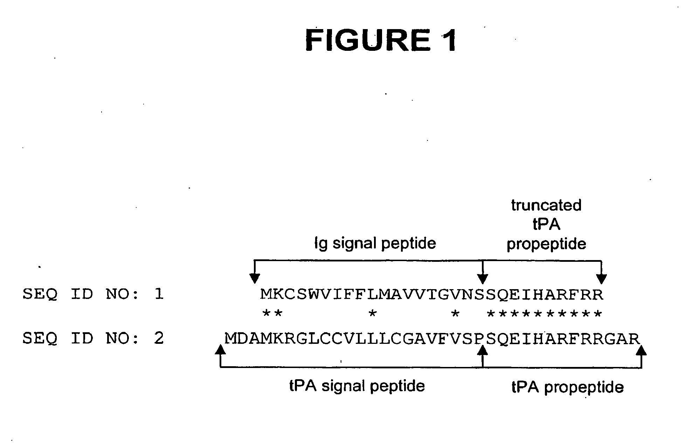

The DNA construct of the present invention comprises a sequence encoding a human tPA propeptide, the carboxyl-terminal extremity of said tPA propeptide consisting of amino acids Arg-Xaa-Arg-Arg. Such a propeptide corresponds to a truncated propeptide lacking the three carboxyl-terminal amino acids of the native human tPA propeptide (see Figure 1). The human tPA propeptide consists of amino acids 23 to 32 of SEQ ID NO:2.

In a preferred embodiment, the DNA construct of the present invention encodes a pre-propeptide comprising SEQ ID NO: 1. In a most preferred embodiment, the pre -propeptide consists of SEQ ID NO: 1.

The terms "comprising", "consisting of", or "consisting essentially of" have distinct meanings. However, each term may be substituted for another herein to change the scope of the invention.

As used herein, the expression "a polypeptide A fused to a polypeptide B" refers to a fusion polypeptide comprising the sequences of polypeptides A and B. wherein the sequence of polypeptide A is located at the amino-terminal extremity of the sequence of polypeptide B within said fusion polypeptide. The term "fused to", as used herein, is not limited to a direct fusion of polypeptides. For example, the cloning strategy may lead to the presence of amino acids between polypeptides A and B. However, a direct fusion of polypeptides is preferred. Methods of constructing DNA constructs comprising sequences encoding fusion polypeptides are well known in the art. For example, methods described in examples 1 to 3 may be used.

Another preferred embodiment of the present invention relates to a DNA construct encoding a fusion polypeptide comprising an IgSP -tPA pre-propeptide fused to a polypeptide of interest.

In accordance with the present invention, the polypeptide of interest may be any polypeptide for which production is desired. For example, the polypeptide of interest may be, e.g., a naturally secreted protein, a normally cytoplasmic protein, a normally transmembrane protein, or a human or a humanized antibody. When the protein of interest is a normally cytoplasmic or a normally transmembrane protein, the protein has preferably been engineered in order to become soluble. The polypeptide of interest may be of any origin. Preferred polypeptides of interest are of human origin.

Preferably, the first amino acid of the polypeptide of interest is not an aliphatic hydrophobic amino acid. Should the first amino acid of the naturally occurring polypeptide of interest be an aliphatic hydrophobic amino acid, the protein of interest has preferably been engineered so that its first amino acid is not an aliphatic hydrophobic amino acid.

In preferred embodiments, the polypeptide of interest is selected from the group consisting of chorionic gonadotropin, follicle-stimulating hormone, lutropin-choriogonadotropic hormone, thyroid stimulating hormone, human growth hormone, interferons (e.g., interferon beta-1a, interferon beta-1b), interferon receptors (e.g., interferon gamma receptor), TNF receptors p55 and p75, interleukins (e.g., interleukin-2, interleukin-11), interleukin binding proteins (e.g., interleukin-18 binding protein), anti-CD11a antibodies, and muteins, fragments, soluble forms, fun ctional derivatives, fusion proteins thereof.

Other preferred polypeptides of interest include, e.g., erythropoletin, granulocyte colony stimulating factor, granulocyte-macrophage colony-stimulating factor, pituitary peptide hormones, menopausal gonadotropin, insulin-like growth factors (e.g., somatomedin-C), keratinocyte growth factor, glial cell line-derived neurotrophic factor, thrombomodulin, basic fibroblast growth factor, insulin, Factor VIII, somatropin, bone morphogenetic protein-2. platelet-derived growth factor, hirudin, epoletin, recombinant LFA-3/IgG1 fusion protein, glucocerebrosidase, and muteins, fragments, soluble forms, functional derivatives, fusion proteins thereof.

Also disclosed herein is a DNA construct comprising a sequence encoding a human tissue-type plasminogen activator propeptide characterized in that its carboxyl-terminal extremity consists of amino acids Arg-Xaa-Arg-Arg.

The tPA propeptide of the present invention may be a human tPA propeptide consisting either of amino acids 24 to 32 of SEQ ID NO: 2 or of amino acids 23 to 32 of SEQ ID NO: 2.

The DNA construct comprising a tPA propeptide in accordance with the invention may further comprise a signal sequence fused to said tPA propeptide.

Any signal peptide currently used In the art for promoting protein secretion may be used in the above DNA construct. Such signal peptides include, e.g., the human growth hormone signal peptide (see, e.g., EP application 01 999 6 52.9), the secretion competent polypeptide disclosed in EP application 00 906 103.7, the human erythropoletin signal peptide, the human albumin signal peptide, the human secreted alkaline phosphatase signal peptide and the rotavirus VP7 glycoprotein signal peptide.

The DNA construct comprising a tPA propeptide may encode a fusion polypeptide comprising said tPA propeptide fused to a polypeptide of interest.

In a preferred embodiment, the DNA construct comprising a sequence encoding an IgSP-tPA pre-propeptide in accordance with the invention is included in a vector.

The term "vector" refers to any carrier of exogenous DNA or RNA that is useful for transferring exogenous DNA to a host cell for replication and/or appropriate expression of the exogenous DNA by the host cell.

In a further preferred embodiment, the vector is an expression vector. An " expression vector" comprises appropriate signals that drive expression in host cells of a polynucleotide inserted in said vector. Preferably, the polynucleotides inserted in said vector encode a polypeptide of interest. The appropriate signals include various regulatory elements, such as enhancers and/or promoters from both viral and mammalian sources. Selectable markers for establishing permanent, stable cell clones expressing the products such as, e.g., a dominant drug selection, are generally included in the expression vectors of the invention, as they are elements that link expression of the drug s election markers to expression of the polypeptide.

In a further preferred embodiment, the vector is a vector for performing gene activation. The gene activation technology is a technology allowing the production of proteins of interest without introducing the gene or the cDNA of interest into the host cell (see e.g., EP patents Nos. 0 505 500 and 0 779 362). For example, the gene activation technology may bypass regulatory DNA sequences set in the "off position" with regulatory DNA sequences set in the "on position" in order to activate the gene of interest. A gene activation vector comprises appropriate signals that drive expression in host cells of a polynucleotide present in said host cell.

In a further preferred embodiment, the vector is a gene therapy vector. Expression vectors that may be used for gene therapy are well known in the art. Preferably, the gene therapy vector is a lentiviral derived vector, which has been shown to be very efficient in the transfer of genes, in particular within the CNS. Oth er well-established viral vectors, such as adenoviral derived vectors, may also be used according to the invention.

A third aspect of the invention relates to a host cell transformed with a DNA construct according to the invention. Many host cells are sui table in accordance with the present invention, such as primary or established cell lines from a wide variety of eukaryotes including plant and animal cells. Preferably, said host cell is an eukaryotic cell. Most preferably, said host cell is a mammalian cell.

For example, suitable host cells include CHO cells, COS cells, CV1 cells, mouse L cells, HT1080 cells, BHK-21 cells, HEK293 cells, NIH-3T3 cells, LM cells, YI cells, NSO and SP2/0 mouse hybridoma cells and the like, Namalwa cells, RPMI-8226 cells, Vero cells, WI-38 cells, MRC-5cells or other immortalized and/or transformed cells.

Preferably, the host cell is a CHO cell, and more preferably a CHO-S cell, described e.g. by Shotwell et al. (1982, J Biol. Chem. 257:2974-2980).

In a third aspect the invention relates to a process for the production of a polypeptide of interest comprising the step of transfecting a host cell with a DNA construct according to the invention.

In a fourth aspect, the invention relates to a process for the production of a poly peptide of interest comprising the step of culturing a host cell in accordance with the invention.

Such processes according to the invention lead to secretion of the protein of interest, which may be harvested from the cell culture supernatant. Depending on the intended use, the cell synthesizing the polypeptide may be the product of the process according to the invention.

In a preferred embodiment, these processes further comprise the step of isolating the polypeptide of interest from the host cells. This step is particularly advantageous and easy to carry out since the protein of interest may simply be isolated from the cell culture supernatant.

These processes may be used in transient, stable, episomal or viral expression systems. In a preferred embodiment, the transfection is stable transfection.

In a fifth aspect, the DNA construct according to the invention is used for producing a polypeptide of interest.

A sixth aspect of the invention relates to fusion polypeptides comprising an IgSP-tPA pre-propeptide according to the invention fused to a polypeptide of interest.

### EXAMPLES

### Example 1: Comparison between the IgSP-tPA pre-propeptide and the human growth hormone signal peptide, the secreted alkaline phosphatase signal peptide, the murine immunogobulin signal peptide.

### 1.1. Constructions.

### 1.1.1 IgSP

The murine IgG µ-heavy chain signal peptide of SEQ ID NO: 3 (IgSP) cloned as follows. Primers of Seq ID Nos. 13 to 20 were incubated with the T4 polynucleotide kinase (Stratagene) for 2h30 at 37°C, and heat inactivated at 75°C for 10 min. The treated primers were ligated using cycle ligation with Pfu Ugase from Stratagene as recommended by the manufacturer in the following cycle conditions:
- 95°C for 1min;
- 40 cycles at 95°C for 30", 57°C for 90", 70°C for 2min
- 70°C for 10min.

The annealed oligos were then purified with QIAquick columns, and PCR amplified with PFU turbo using standard conditions with the primer SEQ ID No 13 and 17 under the following PCR conditions:
- 95 °C 5min
- 30 cycles of 95 °C for 45", 70 °C for 45"; and
- 70 °C for 10min.

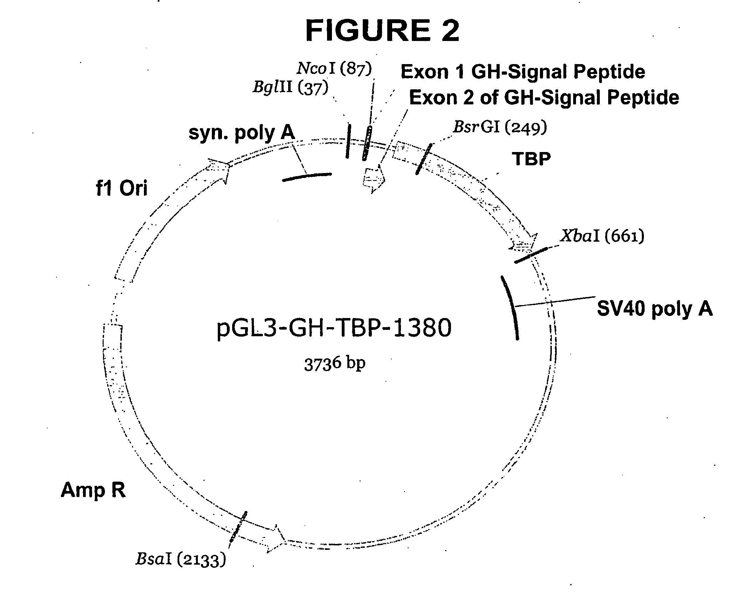

The PCR product was purified and digested with BgI**-**II and BsrGI and cloned into the pGL3-GH-TBPI-1380 vector (Figure 2). The product was sequenced and was found to reflect the expected sequence.

### 1.1.2GH_SP

The human growth hormone signal peptide of SEQ ID NO: 4 (GH_SP) was cloned as follows using primers of SEQ ID No. 21 to 34. The primers were annealed as described above. At the end of the annealing, the product was purified and re -amplified by PCR using the same conditions as above. The PCR product was re-amplified using primers of SEQ ID Nos. 21 and 34. The product was then cloned into the pGL3 -GH-TBPI380 vector at the BgI-II and BsrGI cloning sites.

### 1.1.3. SEAP_SP

The human secreted alkaline phosphatase signal peptide of SEQ ID NO: 5 (SEAP_SP) was cloned using primers of SEQ ID No. 35 to 41. The primers were annealed as described above. At the end of the annealing, the product was purified and re -amplified by PCR using the same conditions as above. The PCR product was re-amplified using primers of SEQ ID Nos. 35 and 40. The product was then cloned into the pGL3-GH-TBPI380 vector at the BgI-II and BsrGI cloning sites.

### 1.1.4. IgSP-tPA

The studied IgSP-tPA pre-propeptide comprised: (i) the murine IgG µ-heavy chain signal peptide fused to (ii) a truncated tPA propeptide that lacks the three carboxyl-terminal amino acids of the native tPA propeptide. This IgSP-tPA pre-propeptide is shown as SEQ ID NO: 1. This IgSP-tPA pre-propeptide was cloned as follows.

Primers of SEQ ID No. 42 to 49 were annealed as described above. At the end of the annealing, the product was purified and re-amplified by PCR using primers of SEQ ID 45 and 49. The purified PCR product was then cloned into the pGL3 -GH-TBP1380 vectors at the BgI-II and BsrGI cloning sites.

A recombinant PCR was performed for deleti ng three amino-acid (GAR) from the known tPA propeptide. The 5' end of the construct was PCR amplified with primers of SEQ ID Nos. 51 and 52, and the 3' end was PCR amplified with primers of SEQ ID Nos. 53 and 54. The full-length IgSP-tPA-TBPI construct was then PCR amplified with primers of SEQ ID Nos. 51 and 54 and the two recombinant products obtained from the previous PCR.

### 1.1.5. Fusion of the above leader sequences to TBPI

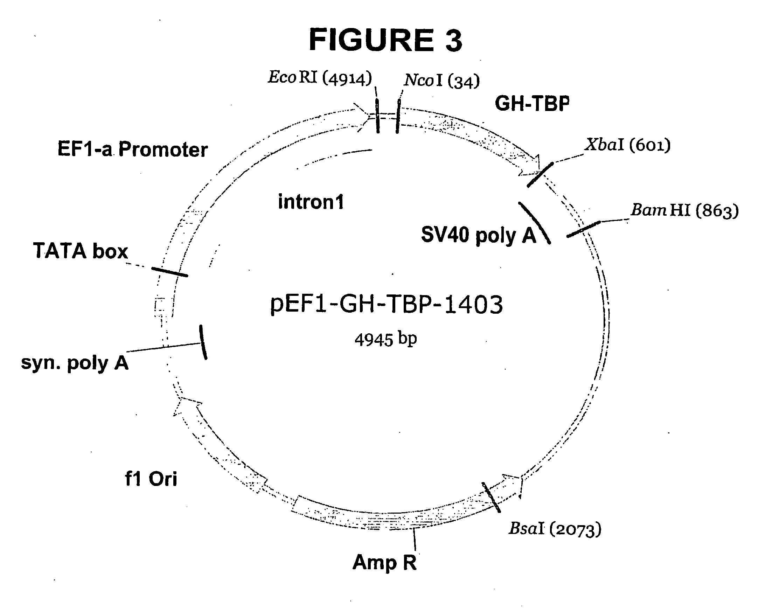

The above leader sequences were fused to the soluble portion of the TNF receptor p55 protein (TBPI, SEQ ID NO: 50). The pEF1-GH-TBPI-1403 vector (Figure 3), which allows expression of TBPI under the Human Elongation Factor 1 (EF1) promoter, was digested by Nco-I and Xba-I. Fragments encoding the leader sequences were subcloned into pEF1-GH-TBPI-1403 as Nco-I or as Bsa-I -Xba-I fragments. The resulting IgSP-TBPI, GH_SP-TBPI SEAP_SP-TBPI and IgSP-tPA constructs were sequenced and were found to reflect the expected sequence.

### 1.2. Measurement of Protein secretion

Each construct was transfected in CHO-DUKX-B11 cells using standard lipid mediated transfection as described in standard laboratory manuals (Maniatis et al., Molecular Cloning: A Laboratory Manual, 2nd, edition, Cold Spring Harbor Laboratory Press). 48hrs after transfection, medium and cells were harvested. Cells were washed twice in PBS and the pellet was lysed on ice using 300µl of Cytobuster buffer (Novagen) in the presence of a protease inhibitor cocktail (Roche). Cellular debries were spinned down by centrifugation at 16 000 g for 10 mn at 4 °C. The supernatant was harvested and kept at -20 °C before being processed. The amount of TBPI released In the supernatant or in intracellular compartment was analyzed by an ELISA. The relative expression of TBPI was measured and reported.

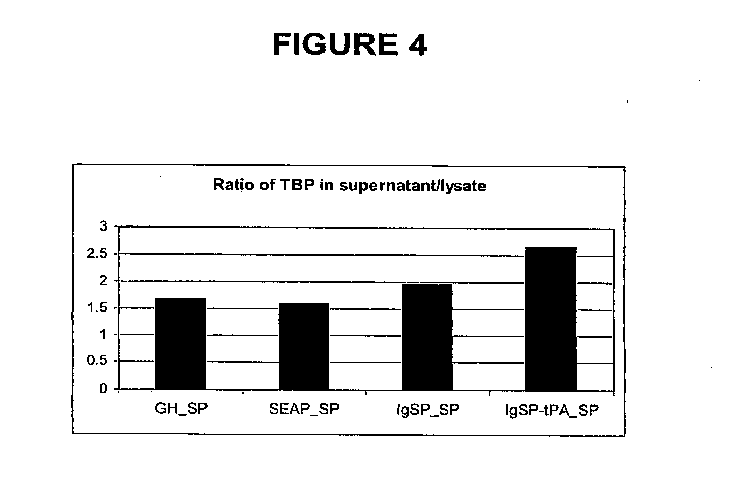

The results of the experiment are shown on Figure 4. The IgSp-tPA signal propeptide is able to boost secretion of TBPI from cells as demonstrated by the increased amount of TBPI detected in the supernatant versus the amount of TBPI detected in intrace lullar compartments. Thus the IgSP-tPA-TBPI construct, comprising TBPI fused to an IgSP-tPA propeptide, increases secretion of TBPI compared to the constructs corresponding to the TBPI protein fused to the IgSP signal peptide, to the secreted alkaline phosphatase signal peptide or to the growth hormone signal peptide.

Accordingly, the IgSP-tPA pre-propeptide ensures a more efficient secretion of TBPI than any other signal peptide fused directly to TBPI without propeptide.

### Example 2: Comparison between the IgSP-tPA pre-propeptide and the tPA pre-propeptide.

### 2.1. Constructions.

### 2.1.1. IgSP-tPA-TBPI

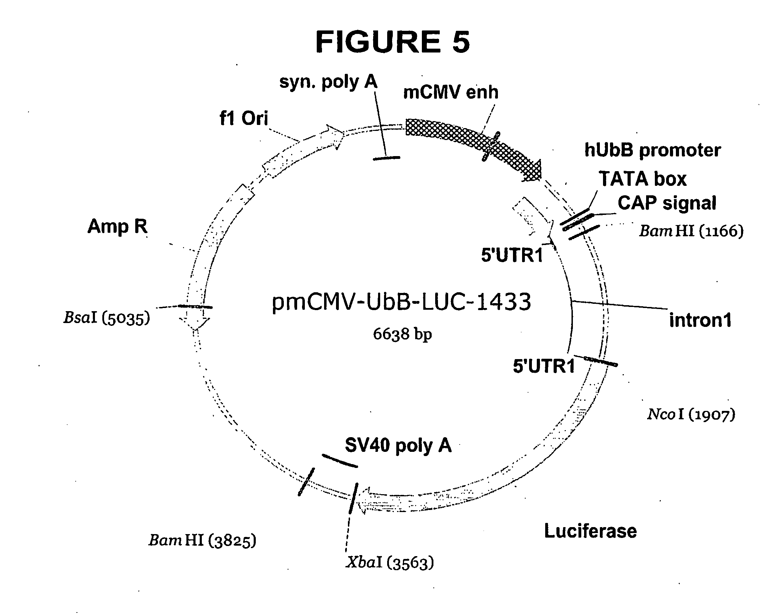

The IgSP-tPA-TBPI construct described in 1.1.5. was digested by Xba-I. The fragment comprising IgSP-tPA-TBPI was cloned into the pmCMV-UbB-LUC-1433 expression vector (Figure 5) digested by Nco-I and Xba-I.

### 2.1.2. tPA-TBPI

A tPA pre-propeptide of SEQ ID NO: 2 comprising: (i) the tPA signal peptide and (if) a truncated tPA propeptide that lacks the three carboxyl -terminal amino acids of the native tPA propeptide was generated as follows.

The human tPA pre-propeptide was cloned using the IgSP-tPA-TBPI construct as a template. A first PCR was performed with primers of SEQ ID No 55 and 56 in order to amplify the tPA propeptide and the 5' end of TBPI. In a second PCR, the PCR product from the first step was extended by re-amplification with primers of SEQ ID Nos. 57, 58 and 56. The PCR product was then cloned into the pGL3-GH-TBPI-1380 vector (Figure 2) digested with Bgl-II and BsrGI. A recombinant PCR was performed as described in 1.1.4. with primers of SEQ ID Nos. 51 to 54 in order to introduce an internal deletion of three amino-acid (GAR) into the known tPA propeptide.

The resulting tPA-TBPI construct was digested by a Bsa-I and Xba-I. The fragment comprising tPA-TBPI was cloned into the pmCMV-UbB-LUC-1433 expression vector digested with Nco-I and Xba-I.

### 2.2. Measurement of protein secretion

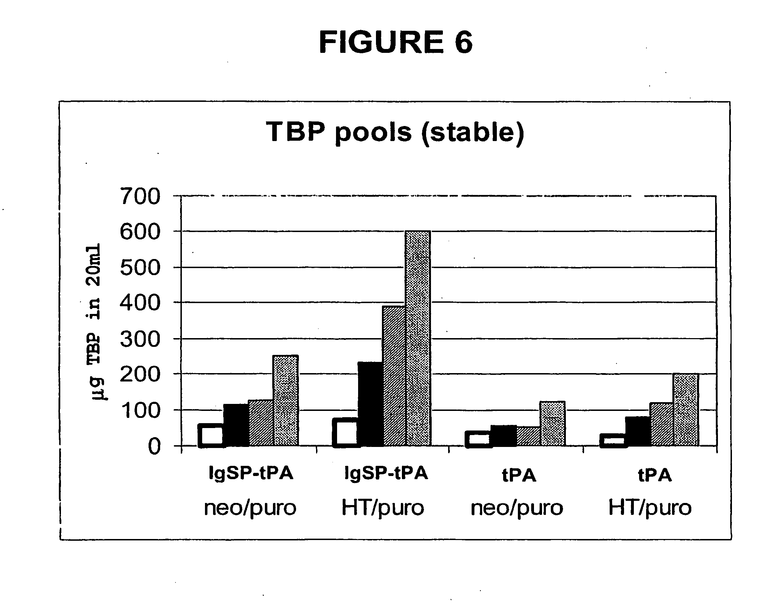

CHO cells were transfected using lipofectamine with the IgSP-tPA-TBPI and the tPA-TBPI constructs. In one series of experiments, the TBPI expression vector was co-transfected with SV40neo and puro vectors for selection. In another series, the TBPI expression vector co-transfected with SV40dhfr and SV40 puro vectors. Pools of stable expressing clones representing at least 100 clones were expanded in different selection medium (600 µg.ml⁻¹ neomycin, 6 µg.ml⁻¹ puromycin, or HT + 6 µg.ml⁻¹ puromycin). Pools were split and cells seeded either in FCS-containing or in serum-free medium. The media were harvested after 48 hrs, and the amount of TBPI released in the supernatant was determined using an ELISA. The result of this experiment is shown in Figure 6. Each box represents a pool. Open boxes and dark boxes represent two different pulses in 10% FCS-containing medium at 37 °C. Striped or squared box represent two different pulses in serum -free medium at 32 °C. The initial number of seeded cells and the pulse periods were similar in each experiment.

Figure 6 shows that in all conditions that were studied, pools of IgSP-tPA-TBPI expressing cells had higher titers of TBPI than pools of tPA-TBPI expressing cells. The results clearly indicate that the IgSP-tPA construct is at least two fold better than tPA construct in terms of quantity of secreted protein that is produced.

Accordingly, the novel combination of the tPA propeptide with the IgSP signal peptide is more efficient at promoting secretion of proteins than the tPA pre -propeptide.

In addition, the sequence of the N-terminal extremity of the TBPI protein secreted from IgSP₄PA-TBPI expressing cells was determined by N-terminal sequencing using Edman degradation. It was found that 100% of the proteins had been cleaved after the last arginine residue of the tPA. Thus the IgSP-tPA pre -propeptide ensures an efficient and reliable processing of polypeptides.

### Example 3: Comparison between the IgSP-tPA pre-propeptide and the interferon gamma receptor signal peptide for production of interferon gamma.

### 3.1. Constructs.

The IgSP pre-pro peptide was fused to a mature Interferon gamma receptor chain protein (IFNAR) and cloned into (i) the mCMV-UbB-LUC-1433 vector (Figure 3): or (ii) a vector comprising the promoter of the mCMV-IE2 gene described in EP application 03100 617.4.

A full-length IFNAR, comprising the native signal peptide, was cloned into (i) the mCMV-UbB-LUC-1433 vector, or (ii) the expression vector comprising the promoter of the mCMV-IE2 gene described in EP application 03 100 617.4.

### 3.2 Measurement of protein secretion

### 3.2.1. Protocol

Constructs were transfected into CHO cells using standard lipid mediated transfection protocols. The secreted proteins were harvested after 48 hrs. A specific Elisa test was used to quantify the amount of IFNAR secreted in the supernatant. The transfections were normalized with a luciferase construct co-transfected with the IFNAR vector. A standard luciferase assay was used as described in standard laboratory manuals (Maniatis et al., Molecular Cloning: A Laboratory Manual, 2nd edition, Cold Spring Harbor Laboratory Press).

### 3.2.1. Constructs comprising the CMV vector

The IgSP-tPA pre-propeptide was about 2.3 fold more efficient in promoting secretion of the IFNAR protein in the supernatant than the native IFNAR signal peptide.

*3.2.2. Constructs comprising the promoter* of *the mCMV-IE2 gene.*

The IgSP-tPA pre-propeptide was about 4.3 fold more efficient in promoting secretion of the IFNAR protein in the supernatant than the native IFNAR signal peptide.

### REFERENCES

Etcheverry et al., ESACT meeting, abstract 01.07/P1.02
Köhne et al (1999) J. Cell. Biochem. 75 :446-461
Loh et al. (1983) Cell. 33:85-93
Nakayama (1997) Biochem. J 327:625-635
Pfeffer and Rothman (1987) Ann.Rev.Biochem. 56:829-852
Seidah and Chretien (1999) Brain Res. 848:45-62
Shotwell et al. (1982) J Biol. Chem. 257:2974-2980
Thomas (2002) Nat Rev Mol Cell Biol. 3:753-766
Watanabe et al (1992) J Biol. Chem. 267:8270-8274

## Claims

1. A DNA construct comprising a sequence encoding an IgSP-tPA pre-propeptide comprising an immunoglobulin signal peptide (IgSP) fused to a tissue-type plasminogen activator (tPA) propeptide consisting of amino acids 23 to 32 of SEQ ID NO: 2.

2. The DNA construct of claim 1, wherein said immunoglobulin signal peptide is a murine immunoglobulin signal peptide.

3. The DNA construct of claim 2, wherein said murine immunoglobulin signal peptide comprises SEQ ID NO: 3.

4. The DNA construct of claim 1, wherein said pre-propeptide comprises SEQ ID. NO:1.

5. The DNA construct of any of claims 1 to 4, wherein said DNA construct encodes a fusion polypeptide comprising said IgSP-tPA pre-propeptide fused to a polypeptide of interest.

6. The DNA construct of any of claims 1 to 5, wherein said DNA construct is included in a vector.

7. The DNA construct of claim 6, wherein said vector is an expression vector.

8. The DNA construct of claim 6, wherein said vector is a vector for performing gene activation.

9. A host cell transformed with the DNA construct of any of claim 1 to 8.

10. The host cell of claim 9, wherein said cell is selected from the group consisting of a CHO cell, a COS cell, a CV1 cell, a mouse L cell, a HT1080 cell, a BHK cell, a HEK293 cell, a NIH-3T3 cell, a LM cell and a YI cell, NS0 and SP2/0 mouse hybridoma and the like, Namalwa, RPMI-8226, Vero, WI-38, MRC-5 and the like.

11. The host cell of claim 9, wherein said cell is a CHO cell.

12. A process for the production of a polypeptide of interest comprising the step of transfecting a host cell with the DNA construct of any of claims 1 to 8.

13. A process for the production of a polypeptide of interest comprising the step of culturing the host cell of any of claims 9 to 11.

14. The process of claim 12 or 13, further comprising the step of isolating the polypeptide of interest from said host cells.

15. The process of any of claims 12 to 14, wherein the transfection is stable transfection.

16. Use of the DNA construct of any of claims 1 to 8 for producing a polypeptide of interest.

17. A fusion polypeptide encoded by the DNA construct of claim 5.

## Patentansprüche

1. DNA-Konstrukt, das eine Sequenz umfasst, die für ein IgSP-tPA Prä-Propeptid kodiert, das ein Immunglobulin-Signalpeptid (IgSP) umfasst, welches mit einem gewebsspezifischen Plasminogen-Aktivator- (tPA) Propeptid, das aus den Aminosäuren 23 bis 32 der SEQ ID NO: 2 besteht, fusioniert ist.

2. DNA-Konstrukt nach Anspruch 1, wobei das Immunglobulin-Signalpeptid ein Immunglobulin-Signalpeptid aus der Maus ist.

3. DNA-Konstrukt nach Anspruch 2, wobei das Immunglobulin-Signalpeptid die SEQ ID NO: 3 umfasst.

4. DNA-Konstrukt nach Anspruch 1, wobei das Prä-Propeptid die SEQ ID NO:1 umfasst.

5. DNA-Konstrukt nach einem der Ansprüche 1 bis 4, wobei das DNA-Konstrukt für ein Fusionspolypeptid kodiert, welches das IgSP-tPA Prä-Propeptid, das mit einem Polypeptid von Interesse fusioniert ist, umfasst.

6. DNA-Konstrukt nach einem der Ansprüche 1 bis 5, wobei das DNA-Konstrukt in einem Vektor enthalten ist.

7. DNA-Konstrukt nach Anspruch 6, wobei der Vektor ein Expressionsvektor ist.

8. DNA-Konstrukt nach Anspruch 6, wobei der Vektor ein Vektor zur Durchführung von Genaktivierung ist.

9. Wirtszelle, die mit dem DNA-Konstrukt nach einem der Ansprüche 1 bis 8 transformiert ist.

10. Wirtszelle nach Anspruch 9, wobei die Zelle aus der Gruppe bestehend aus einer CHO-Zelle, einer COS-Zelle, einer CV1-Zelle, einer Maus-L-Zelle, einer HT1080-Zelle, einer BHK-Zelle, einer HEK293-Zelle, einer NIH-3T3-Zelle, einer LM-Zelle und einer YI-Zelle, NSO und SP2/0 Maus-Hybridom und dergleichen, Namalwa, RPMI-8226, Vero, WI-38, MRC-5 und dergleichen ausgewählt ist.

11. Wirtszelle nach Anspruch 9, wobei die Zelle eine CHO-Zelle ist

12. Verfahren zur Herstellung eines Polypeptids von Interesse, welches den Schritt Transfizieren einer Wirtszelle mit dem DNA-Konstrukt nach einem der Ansprüche 1 bis 8 umfasst.

13. Verfahren zur Herstellung eines Polypeptids von Interesse, welches den Schritt Kultivieren der Wirtszelle nach einem der Ansprüche 9 bis 11 umfasst.

14. Verfahren nach Anspruch 12 oder 13, welches weiterhin den Schritt Isolieren des Polypeptids von Interesse aus den Wirtszellen umfasst.

15. Verfahren nach einem der Ansprüche 12 bis 14, wobei die Transfektion eine stabile Transfektion ist.

16. Verwendung des DNA-Konstrukts nach einem der Ansprüche 1 bis 8 zur Herstellung eines Polypeptids von Interesse.

17. Fusionspolypeptid, das von dem DNA-Konstrukt nach Anspruch 5 kodiert wird.

## Revendications

1. Construction d'ADN, comprenant une séquence qui code un pré-propeptide IgSP-tPA comprenant un peptide signal d'immunoglobuline IgSP fusionné à un propeptide d'activateur tissulaire du plasminogène tPA, constitué des acides aminés n° 23 à 32 de la Séquence N° 2.

2. Construction d'ADN conforme à la revendication 1, pour laquelle ledit peptide signal d'immunoglobuline est un peptide signal d'immunoglobuline murin.

3. Construction d'ADN conforme à la revendication 2, pour laquelle ledit peptide signal d'immunoglobuline murin comprend la Séquence N° 3.

4. Construction d'ADN conforme à la revendication 1, pour laquelle ledit pré-propeptide comprend la Séquence N° 1.

5. Construction d'ADN conforme à l'une des revendications 1 à 4, laquelle construction d'ADN code un polypeptide de fusion comprenant ledit pré-propeptide IgSP-tPA fusionné à un polypeptide présentant de l'intérêt.

6. Construction d'ADN conforme à l'une des revendications 1 à 5, laquelle construction d'ADN est incluse dans un vecteur.

7. Construction d'ADN conforme à la revendication 6, pour laquelle ledit vecteur est un vecteur d'expression.

8. Construction d'ADN conforme à la revendication 6, pour laquelle ledit vecteur est un vecteur conçu pour réaliser l'activation d'un gène.

9. Cellule hôte transformée avec une construction d'ADN conforme à l'une des revendications 1 à 8.

10. Cellule hôte conforme à la revendication 9, laquelle cellule est choisie dans l'ensemble formé par une cellule CHO, une cellule COS, une cellule CV1, une cellule L de souris, une cellule HT1080, une cellule BHK, une cellule HEK293, une cellule NIH-3T3, une cellule LM et une cellule YI, les hybridomes de souris NSO et SP2/0 et similaires, et les cellules Namalwa, RPMI-8226, Vero, WI-38, MRC-5 et similaires.

11. Cellule hôte conforme à la revendication 9, laquelle cellule est une cellule CHO.

12. Procédé de production d'un polypeptide présentant de l'intérêt, lequel procédé comporte une étape de transfection d'une cellule hôte avec une construction d'ADN conforme à l'une des revendications 1 à 8.

13. Procédé de production d'un polypeptide présentant de l'intérêt, lequel procédé comporte une étape de culture d'une cellule hôte conforme à l'une des revendications 9 à 11.

14. Procédé conforme à la revendication 12 ou 13, qui comporte en outre une étape d'isolement du polypeptide présentant de l'intérêt à partir desdites cellules hôtes.

15. Procédé conforme à l'une des revendications 12 à 14, dans lequel la transfection est une transfection stable.

16. Utilisation d'une construction d'ADN conforme à l'une des revendications 1 à 8 en vue de la production d'un polypeptide présentant de l'intérêt.

17. Polypeptide de fusion, codé par une construction d'ADN conforme à la revendication 5.
